# EUROPEAN PATENT SPECIFICATION

(11) **EP 0 281 219 B1**
(45) Date of publication and mention of the grant of the patent: **14.07.1993**
(21) Application number: 88300292.5
(22) Date of filing: 14.01.1988
(51) Int. Cl.: A61N 1/39, A61N 1/05

(54) **Cardiac defibrillator**
Herzdefibrillator
Défibrillateur cardiaque

(30) Priority: 14.01.1987 US 3358
(43) Date of publication of application: 07.09.1988
(73) Proprietor: MEDTRONIC, INC., Minneapolis, Minnesota 55432-3576 (US)
(72) Inventor: Mehra, Rahul, Stillwater Minnesota 55082 (US); Combs, William, Minneapolis Minnesota 55410 (US)
(74) Representative: Tomlinson, Kerry John

(56) References cited:
- EP-A- 0 117 972
- FR-A- 2 561 929
- GB-A- 2 070 435
- GB-A- 2 083 363
- US-A- 3 211 154
- US-A- 3 224 447
- US-A- 3 241 555
- US-A- 4 010 755
- US-A- 4 026 302
- US-A- 4 498 478
- US-A- 4 662 377
- Cardiovascular research, vol.18, 1984, John C.Schuder et al: "Defibrillation of 100 kg calces with asymetrical, bidirectional, rectangular pulses", pages 419-426

## Description

### BACKGROUND OF THE INVENTION

This invention relates to the control of cardiac arrhythmias and particularly to terminating fibrillation in the heart.

Ventricular fibrillation is characterized by the random depolarization of individual fibers of the heart which greatly reduces the cardiac output of the heart and leads to death within minutes of onset. Conventional external treatment for fibrillation calls for the application of an electric shock supplied by a pair of paddles across the chest of the patient which simultaneously depolarizes all of the cardiac muscle fibers. This process permits a resynchronization of the ventricular muscle fibers.

Implantable defibrillator systems have been proposed for use in patients susceptible to sudden death syndrome. Traditionally such systems have comprised an implanted pulse generator coupled to a plurality of electrodes located in and around the heart. Such implantation techniques require a thorocotomy to place the electrodes. However, the most fundamental problem associated with implantable defibrillation is the high energy required to successfully defibrillate the heart.

One early attempt to produce an electrode system suitable for use in an automatic implantable defibrillator is illustrated in U.S. Patent No. 3,942,536. In this system, a single right ventricular endocardial lead is used having one set of electrodes at its distal tip for location in the apex of the right ventricle, and a second set of electrodes spaced from the set of electrodes on the distal tip a sufficient distance to place them outside the heart, in the superior vena cava. Other endocardial ventricular defibrillation lead systems are illustrated in U.S. Patent No. 3,857,398 issued to Rubin and in U.S. Patent No. 4,355,646 issued to Kallok.

Experience with these lead systems has shown that the energy required to defibrillate the heart utilizing a single pair of electrodes, while significantly less than that required by use of an external defibrillator, is still sufficiently large to make construction of a battery powered automatic implantable defibrillator difficult. Additionally, the small electrode areas required by catheter mounted systems have been shown to increase the risk of tissue damage because of the increased current density present at the electrode sites.

In an effort to overcome this problem, electrode systems have been proposed such as that shown in U.S. Patent No. 4,030,509 to Heilman which shows a collection of large surface area electrodes. One set of electrodes is applied to the apex of the heart, a second set is applied to the atria of the heart. As an alternative, it has been suggested that a superior vena cava electrode on an endocardial lead may also be used in conjunction with the large area electrode applied directly to the apex of the heart. One problem associated with the use of epicardial patch electrodes on the heart is that the surgery to attach the electrodes is highly invasive, and therefore undesirable.

Other large surface area electrodes for application to the human heart are disclosed in U.S. Patent No. 4,291,707 issued to Heilman et al, which discloses electrodes fabricated of metallic mesh, sandwiched between two layers of chemically inert electrically insulative material.

Recently, it has been proposed that rather than delivering electrical energy between electrodes located in the apex of the heart and electrodes located on or in the superior vena cava or atrium of the heart that a return to application of electrical energy transversely across the heart is desirable For example, in published European Patent Application Publication No. 0 095 726 by the Purdue Research Foundation, it is proposed that four epicardial mesh electrodes be arranged orthogonally around the heart and that defibrillation be accomplished using two sequential orthogonal defibrillation pulses.

A large portion of the early work on automatic implantable and external defibrillators was performed with stimulators which provided a single monophasic pulse, or a symmetrical biphasic pulse.

The article by John C. Schuder in Cardiovascular Research vol.18, 1984 pages 419 to 426 discloses external defibrillation of calves using externally applied asymmetrical energy pulses.

The present invention is directed toward apparatus for defibrillating the heart utilizing a novel asymmetric biphasic stimulation pulse.

The novel defibrillation waveform is produced by the truncated discharge of a capacitor in conjunction with polarity reversal at the electrode site. This procedure produces an asymmetric biphasic pulse and permits a biphasic output circuit to be constructed with only a single output capacitor.

In the preferred embodiment, a combination of electrode placement and size have been optimized to produce a system capable of defibrillation at lower energies than have been heretofore possible. It appears that the electrode construction and placement achieves sufficient spatial isolation to minimize the risk of myocardial damage. The electrode system also has the major advantage of not requiring a thoracotomy for electrode placement.

The electrode system comprises a single catheter having one or more electrode areas placed at the distal end for location in the apex of the ventricle. A second set of catheter-borne electrodes is located outside of the atrium of the heart to reduce the possibility of generating atrial fibrillation during ventricular defibrillation. The third electrode of the system is placed subcutaneously laterally near the heart, but outside the rib cage of the patient. This electrode system may be implanted under local anaesthetic, and does not require a thorocotomy or entry into the chest cavity of the patient.

Experimentation with this electrode configuration has shown a marked reduction in the amount of energy required to defibrillate the heart when used with an asymmetric biphasic stimulation pulse in comparison to the energies required to defibrillate utilizing sequential stimulation pulses at these electrode sites.

### BRIEF DESCRIPTION OF THE DRAWINGS

Fig. 1 is a simplified cross-section of the heart showing the location of the electrodes.

Fig. 2 is a schematic diagram of a circuit for generating the asymmetric biphasic waveform of Fig. 3.

Fig. 3 is a waveform diagram showing the asymmetric biphasic stimulation waveform in comparison with a prior art biphasic waveform.

Fig. 4 is a table comparing experimental results of the present lead system and asymmetric biphasic waveform with the prior art sequential monophasic waveform.

### DETAILED DESCRIPTION OF THE ILLUSTRATIVE EMBODIMENT

The invention described herein is based upon laboratory work reported in tabular form within the description. The electrode system utilized within the heart is similar to a Medtronic® 6880 lead which is the subject of U.S. Patent No. 4,355,646, issued to Kallok et al.

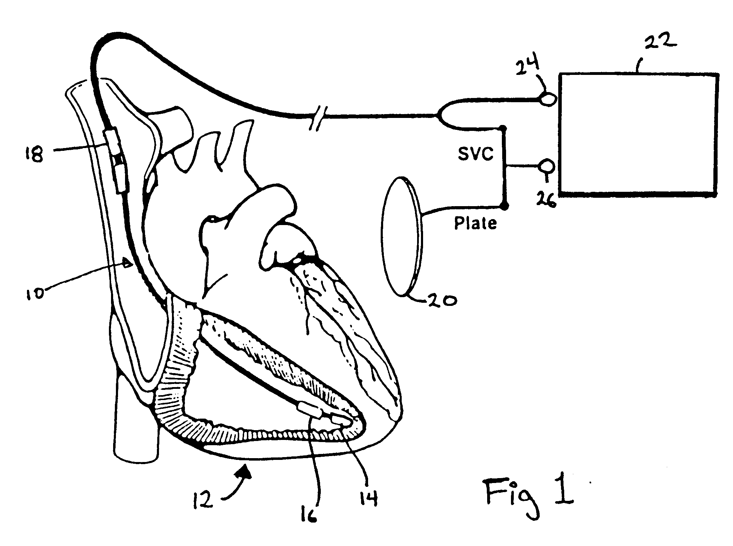

As shown in Fig. 1, a catheter 10 may be inserted into the heart generally shows as 12. A number of catheter-borne electrodes are shown in the figure, and include a pacing tip 14 at the distal end of the electrode with a ring electrode 16 spaced apart in the ventricle. In a similar fashion, a ring electrode is shown outside of the heart at 18. The electrode system also comprises a subcutaneous disk or plate electrode shown as 20 in the figure.

In operation, the distal ring electrode is connected to a conductor which is brought out through a terminal block and is electrically connected to the defibrillator 22 through a first electrode terminal 24. The subcutaneous plate electrode 20 is electrically interconnected with the proximal ring electrode 18, and are electrically connected to the defibrillator through a second terminal 26.

Experimental laboratory work has indicated that this arrangement is preferred for defibrillating the heart, since it results in a substantial reduction in the amount of energy required to defibrillate the heart.

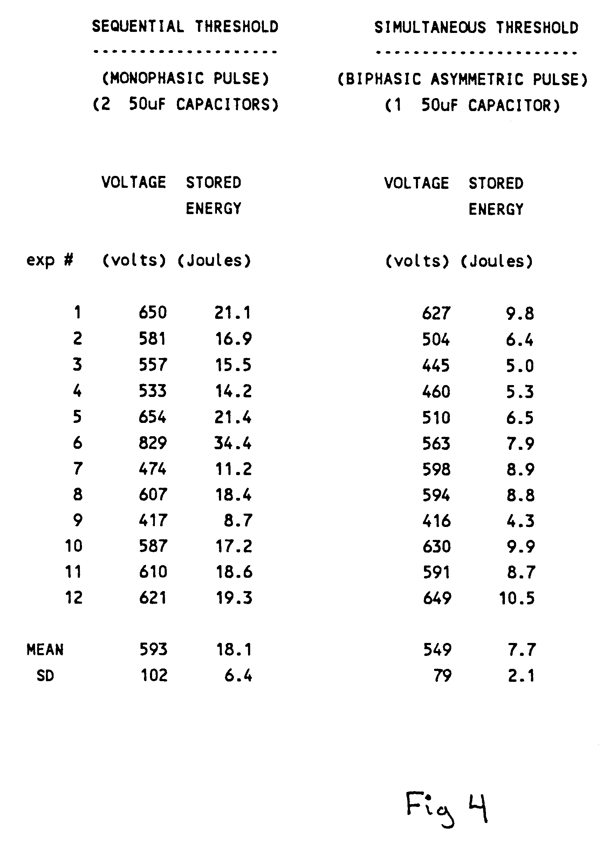

The table of Fig. 4 characterizes the improvement obtained with the present invention. In the experiment, the lead system was implanted as described, and defibrillation thresholds were measured using two sequential monophasic pulse waveforms and one biphasic asymmetrical pulse waveform disclosed herein. The mean defibrillation threshold for the prior art waveform was 593 volts, while defibrillation was achieved at a lower mean voltage of 549 volts for the asymmetric biphasic waveform. Most significantly, the mean stored energy required to defibrillate is only 7.7 joules with the asymmetric biphasic waveform as compared with over 18 joules with the prior art monophasic pulse waveform.

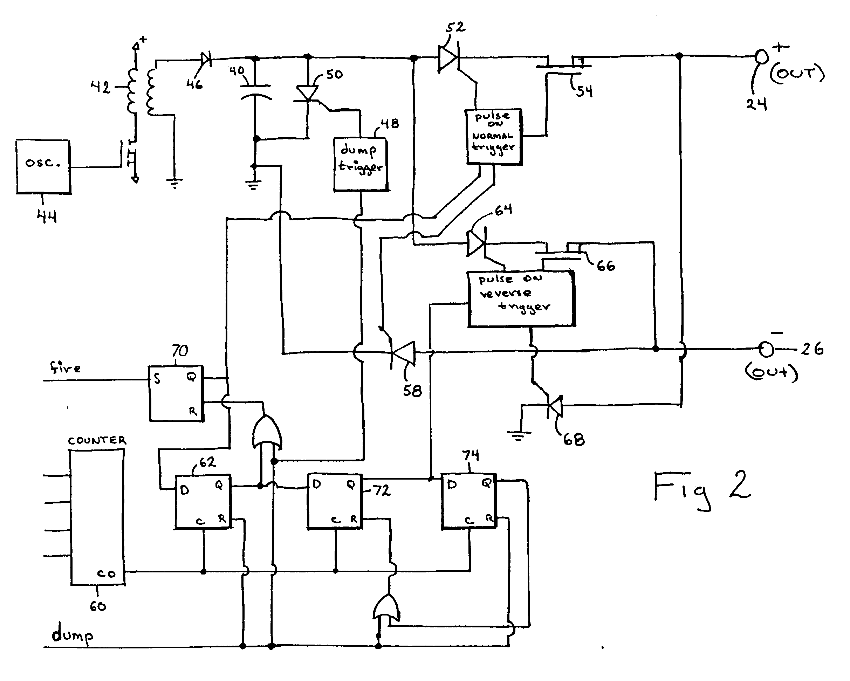

The asymmetric biphasic waveform is produced by the circuit of Fig. 2.

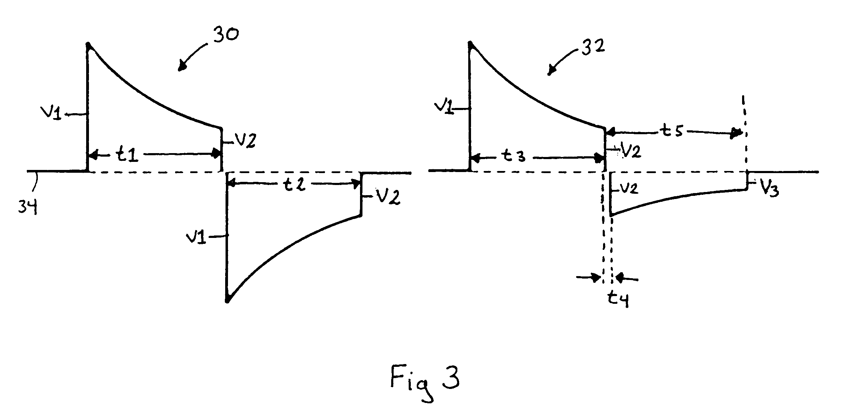

Turning to Fig. 3, there is shown a prior art waveform designated generally 30 shown in proximity to the asymmetric biphasic waveform generated by the circuitry of Fig. 2 shown generally as 32. In the prior art system, a first capacitor is coupled to an electrode system and discharged from a first voltage V1 through an electrode system representing a resistive impedance to a second lower voltage level V2. After polarity reversal, a second capacitor, which has likewise been charged to voltage V1, is discharged through the lead system to the second voltage level V2. This waveform, although efficacious, requires multiple capacitors, and results in a substantially symmetric waveform about the base line 34.

In contrast, the circuitry of Fig. 2 generates an asymmetric waveform 32 through the discharge of a single capacitor from a first voltage V1 to a second voltage V2. After polarity reversal, the electrode system continues the discharge from V2 down to a third voltage V3. As can be seen from the figure, this does not result in a symmetric waveform about the base line 34, but rather results in an unbalanced or asymmetric waveform. The experimental work performed with the asymmetric waveform involved pulse durations for each phase of the waveform (t3, t5) which where equal and varied between 4 and 5 milliseconds. In a similar fashion, the defibrillation threshold was found to correspond to a V1 voltage of between 400 and 700 volts.

The asymmetrical biphasic pulse waveform of Fig. 3 may be generated with a circuit as shown in Fig. 2, wherein the electrode system is coupled to a terminal network comprising a first 24 and second 26 terminals. The energy storage means may comprise one or more capacitors shown in the schematic as 40 but preferably only a single capacitor is used. The capacitor is charged from a transformer 42 coupled oscillator circuit 44. The oscillating voltage produced by the secondary winding of the transformer is rectified by diode 46 for storage in capacitor 40. Should discharge of capacitor 40 be required by the system, dump trigger 48 will control the gate of SCR 50 to provide a discharge path for the energy stored in capacitor 40 to ground.

When defibrillation of the heart is required, detection circuitry (not shown) generates a first input on the set input of flip-flop 70. The Q output turns on SCR52 and SCR58 and FET 54 initiating the leading edge V1 of the output waveform 32 between terminals 24 and 26. Counter preset input applied to counter 60 determines the t3 time duration of waveform 32. When the counter 60 times out, the carry out signal sets flip-flop 62 which resets flip-flop 70. This operation terminates the t3 time period.

The counter 60 is then preset to time out the t4 separation period. Upon time out, the carry out signal of the counter 60 sets the Q output of flip-flop 72 which turns on SCR 64, FET 66 and SCR 68.

The counter is next preset to time out the t5 pulse duration period. When the counter times out the t5 period, the carry out signal of the counter sets the Q output of flip-flop 74, thus resetting flip-flop 72, and thus terminating the t5 period of the pulse waveform 32.

At the conclusion of this timing cycle, the dump signal may reset all the flip-flops and discharge capacitor 40 and the charge oscillator may begin to recharge the energy storage capacitor 40 as previously described in preparation for the next defibrillation pulse if additional defibrillation shocks are required.

## Claims

1. An implantable cardiac defibrillator comprising pulse generating means for generating a defibrillating pulse and terminal means including first and second terminals (24,26) adapted to be connected to a plurality of implantable electrodes, said pulse generating means including energy storage means (40) and circuit means (60,70,54,72,66) arranged to discharge said energy storage means between said first and second terminals for a first predetermined time period (t3), followed by reversing the polarity of said energy storage means with respect to said first and second terminals, then discharging said energy storage means between said first and second terminals for a second predetermined time period (t5), forming an asymmetrical biphasic pulse waveform.

2. An implantable defibrillator as claimed in claim 1 wherein said circuit means is arranged to discharge said energy storage means between said first and second terminals from a first voltage (v1) to a second voltage (v2); interrupt the discharge of said energy storage means; reverse the polarity of said first and second terminals; and resume discharge of said energy storage means from said second voltage (v2) to a third voltage (v3).

3. An implantable defibrillator as claimed in claim 1 or 2 wherein said energy storage means (40) comprises a single capacitor.

4. An implantable defibrillator as claimed in claim 1, 2 or 3 including three electrodes (16,18,20) connected to said terminal means, one of said electrodes (16) being connected to said first terminal (24) and the other two (18,20) being connected together and to said second terminal (26).

5. An implantable defibrillator as claimed in claim 4 comprising an intravenous lead (10) having a first said electrode (16) at a distal end thereof and a second said electrode (18) spaced apart from said first electrode, and a plate electrode (20) of a construction suitable for subcutaneous implantation, said first electrode being connected to said first terminal and said second electrode and said plate electrode being connected together and to said second electrode.

6. An implantable defibrillator as claimed in claim 5 wherein said first electrode (16) is adapted to be positioned at the apex of the right ventricle of a heart and the second electrode (18) in the superior vena cava.

## Patentansprüche

1. Implantierbare Herzentflimmerungsanordnung mit einer Impulserzeugungseinrichtung zum Erzeugen eines Entflimmerungsimpulses und mit Anschlußeinrichtungen mit einem ersten und einem zweiten Anschluß (24, 26), die so ausgebildet sind, daß sie mit mehreren implantierbaren Elektroden verbunden werden können, welche Impulserzeugungseinrichtung eine Energiespeichereinrichtung (40) und eine Schaltungseinrichtung (60, 70, 54, 72, 66) aufweist, die so ausgebildet sind, daß sie die Energiespeichereinrichtung über den ersten und den zweiten Anschluß während einer ersten vorgegebenen Zeitspanne (t3) entladen, gefolgt von einer Umkehr der Polarität der Energiespeichereinrichtung hinsichtlich des ersten und des zweiten Anschlusses, um dann die Energiespeichereinrichtung über den ersten und zweiten Anschluß während einer zweiten vorgegebenen Zeitspanne (t5) zu entladen, um einen asymmetrischen, zweiphasigen Impulssignalzug zu bilden.

2. Implantierbare Entflimmerungsanordnung nach Anspruch 1, bei der die Schaltungseinrichtung so ausgebildet ist, daß sie die Energiespeichereinrichtung über den ersten und zweiten Anschluß ausgehend von einer ersten Spannung (v1) auf eine zweite Spannung (v2) entlädt, die Entladung der Energiespeichereinrichtung unterbricht, die Polarität des ersten und des zweiten Anschlusses umkehrt und die Entladung der Energiespeichereinrichtung ausgehend von der zweiten Spannung (v2) zu einer dritten Spannung (v3) wieder aufnimmt.

3. Implantierbare Entflimmerungsanordnung nach Anspruch 1 oder Anspruch 2, bei der die Energiespeichereinrichtung (40) einen einzigen Kondensator aufweist.

4. Implantierbare Entflimmerungsanordnung nach einem der Ansprüche 1, 2 oder 3, mit drei mit der Anschlußeinrichtung verbundenen Elektroden (16, 18, 20), wobei eine der Elektroden (16) mit dem ersten Anschluß (24) verbunden ist und die beiden anderen Elektroden (18, 20) miteinander und mit dem zweiten Anschluß (26) verbunden sind.

5. Implantierbare Entflimmerungsanordnung nach Anspruch 4, mit einer intravenösen Zuleitung (10) mit der ersten Elektrode (16) am entfernten Ende und der zweiten Elektrode (18), die von der ersten Elektrode beabstandet ist, und einer plattenförmigen Elektrode (20) mit einem Aufbau, der für subkutane Implantation geeignet ist, wobei die erste Elektrode mit dem ersten Anschluß verbunden ist und die zweite Elektrode und die plattenförmige Elektrode miteinander und mit dem zweiten Anschluß verbunden sind.

6. Implantierbare Entflimmerungsanordnung nach Anspruch 5, bei der die erste Elektrode (16) so ausgebildet ist, daß sie an der Spitze der rechten Kammer eines Herzens angeordnet werden kann und die zweite Elektrode (18) in der oberen Hohlvene.

## Revendications

1. Défibrillateur cardiaque implantable comprenant des moyens générateurs d'impulsions servant à produire une impulsion de défibrillation, et des moyens formant bornes comprenant des première et seconde bornes (24,26) adaptées pour être raccordées à une pluralité d'électrodes implantables, lesdits moyens générateurs d'impulsions comprenant des moyens de stockage d'énergie (40) et des moyens formant circuit (60,70,54,72,66) disposés de manière à décharger lesdits moyens de stockage d'énergie entre lesdites première et seconde bornes pendant un premier intervalle de temps prédéterminé (t3), suivi par une inversion de la polarité desdits moyens de stockage d'énergie par rapport auxdites première et seconde bornes, puis déchargement desdits moyens de stockage d'énergie entre lesdites première et seconde bornes pendant un second intervalle de temps prédéterminé (t5), avec formation d'une forme d'onde d'impulsion biphasée dissymétrique.

2. Défibrillateur implantable suivant la revendication 1, dans lequel lesdits moyens formant circuit sont agencés de manière à décharger desdits moyens de stockage d'énergie entre lesdites première et seconde bornes depuis une première tension (v1) jusqu'à une seconde tension (v2); interrompre la décharge desdits moyens de stockage d'énergie; inverser la polarité desdites première et seconde bornes; et reprendre la décharge desdits moyens de stockage d'énergie depuis ladite seconde tension (v2) jusqu'à une troisième tension (v3).

3. Défibrillateur implantable suivant la revendication 1 ou 2, dans lequel lesdits moyens de stockage d'énergie (40) comprennent un seul condensateur.

4. Défibrillateur implantable suivant la revendication 1, 2 ou 3, comprenant trois électrodes (16,18,20) raccordées auxdits moyens formant bornes, l'une desdites électrodes (16) étant raccordée à ladite première borne (24) et les deux autres (18,20) étant raccordées ensemble et à ladite seconde borne (26).

5. Défibrillateur implantable suivant la revendication 4, comprenant un conducteur intraveineux (10) possédant ladite première électrode (16) sur son extrémité distale et ladite seconde électrode (18) distante de ladite première électrode, et une électrode en forme de plaque (20) possédant une construction convenant pour une implantation sous-cutanée, ladite première électrode étant raccordée à ladite première borne, et ladite seconde électrode et ladite électrode en forme de plaque étant raccordées ensemble et à ladite seconde borne.

6. Défibrillateur implantable suivant la revendication 5, dans lequel ladite première électrode (16) est adaptée pour être positionnée au sommet du ventricule droit d'un coeur, et la seconde électrode (18) dans la veine cave supérieure.
